# EUROPEAN PATENT SPECIFICATION

(11) **EP 2 211 631 B1**
(45) Date of publication and mention of the grant of the patent: **25.04.2012**
(21) Application number: 08847513.2
(22) Date of filing: 30.10.2008
(51) Int. Cl.: A23F 3/16, A23F 3/18, A23F 3/30

(54) **PROCESS FOR MANUFACTURING LEAF TEA**
VERFAHREN ZUR HERSTELLUNG VON BLATTTEE
PROCÉDÉ DE PRÉPARATION DE THÉ EN FEUILLES

(30) Priority: 05.11.2007 EP 07119988; 05.11.2007 EP 07119984; 12.11.2007 EP 07120448; 12.11.2007 EP 07120447; 19.12.2007 EP 07123586; 07.02.2008 EP 08151155; 02.10.2008 EP 08165775; 02.10.2008 EP 08165776
(43) Date of publication of application: 04.08.2010
(73) Proprietor: Unilever PLC, A Company Registered in England and Wales under Company no. 41424, London, Greater London EC4Y 0DY (GB); Unilever N.V., 3013 AL Rotterdam (NL)
(72) Inventor: COLLIVER, Steven, Peter, Sharnbrook Bedfordshire MK44 1LQ (GB); SHARP, David, George, Sharnbrook Bedfordshire MK44 1LQ (GB)
(74) Representative: Keenan, Robert Daniel
(86) International application number: PCT/EP2008/064716
(87) International publication number: WO 2009/059926

(56) References cited:
- EP-A- 1 062 941
- EP-A- 1 365 657
- GB-A- 968 423
- GB-A- 191 011 310
- JP-A- 11 056 243
- JP-A- 2006 131 512
- US-A1- 2004 001 862
- US-A1- 2004 180 077
- US-A1- 2006 210 653
- FUKUDA M ET AL: "Manufacture of non-polymer catechin composition from tea leaves, by adding water-soluble acid to water-soluble constituent extracted from green tea leaf, reducing pH, adsorbing with synthetic adsorbent material and eluting" WPI/THOMSON,, 1 January 1900 (1900-01-01), XP002462468

## Description

### TECHNICAL FIELD OF THE INVENTION

The present invention relates to leaf tea. In particular, the present invention relates to green leaf tea made from tea leaves from which juice has been expressed.

### BACKGROUND TO THE INVENTION

Green tea is a popular beverage which has been consumed in China and Japan for many hundreds of years. Recently, extensive laboratory research and epidemiologic studies have shown that compounds present in green tea (particularly catechins) may reduce the risk of a variety of illnesses. Furthermore, catechins have been shown to suppress accumulation of visceral fat and so may be useful in controlling bodyweight and bodyshape (see, for example, T. Nagao et al., "Tea Catechins Suppress Accumulation of Body Fat in Humans", J. Oleo. Sci., 2001, 50(9), pp.717-728). These studies, along with the increasing complexity of the consumer's palate have led to growth in the consumption of green tea, even in markets (such as the USA and Western Europe) where there is no tradition of green tea consumption.

Although, some of the health benefits of tea may be apparent at consumption rates as low as three cups per day (see, for example, U.Peters et al., "Does tea affect cardiovascular disease? A meta-analysis.", American Journal of Epidemiology, 2001, 154, pp.495-503), many individuals do not even achieve this modest consumption rate on a long term basis. Furthermore, tea beverages are less convenient to prepare than beverages prepared from non-tea-based beverage precursors, such as instant coffee, owing to the relatively slow rate of infusion of tea leaves and slow rate of dissolution of tea powders.

Thus we have recognised that there is a need to provide beverage precursor in a form which is both convenient for everyday use and which may allow a consumer to obtain the necessary intake of catechins from a fewer number of beverages than would need to be prepared from conventional beverage precursors.

WO 2008/012280 (Unilever) discloses a beverage precursor comprising tea material and food-grade additive, the beverage precursor being present in an amount wherein if the beverage precursor is contacted with 250 ml water for 2 minutes at 90°C a beverage would be produced comprising catechins in an amount of between 0.05 and 2% by weight of the beverage. The beverage precursor of WO 2008/012280 is provided in an amount that allows for consumers to meet the daily intake of catechins required to achieve health benefits, whilst requiring shorter time to prepare a beverage and/or preparation of fewer beverages per day compared with conventional beverage precursors. A method for manufacturing such a precursor from fresh tea leaf is also disclosed in WO 2008/012280 and comprises a specific maceration step involving a rotorvane and at least one CTC process.

We have found that beverage precursors comprising leaf tea with a high efficiency of catechin delivery, such as those disclosed in WO 2008/012280 can be manufactured from leaf residue which remains after pressing juice from tea leaves. This is surprising since removing juice from leaves would be expected to decrease the total catechin content of the leaves.

### TESTS AND DEFINITIONS

### Tea

"Tea" for the purposes of the present invention means material from *Camellia sinensis* var. *sinensis* and/or *Camellia sinensis* var. *assamica.* Especially preferred is material from var. *assamica* as this has a higher level of tea actives than var. *sinensis.* Thus it is preferred that the tea comprises at least 90% by weight of material from *Camellia sinensis* var. *assamica,* more preferably at least 95% and most preferably from 98 to 100%.

"Leaf tea" for the purposes of this invention means a tea product that contains tea leaves and/or stem in an uninfused form, and that has been dried to a moisture content of less than 30% by weight, and usually has a water content in the range 1 to 10% by weight (i.e. "made tea").

"Green tea" refers to substantially unfermented tea. "Black tea" refers to substantially fermented tea. "Oolong tea" refers to partially fermented tea.

"Fermentation" refers to the oxidative and hydrolytic process that tea undergoes when certain endogenous enzymes and substrates are brought together, e.g., by mechanical disruption of the cells by maceration of the leaves. During this process colourless catechins in the leaves are converted to a complex mixture of yellow and orange to dark-brown polyphenolic substances.

"Fresh tea leaves" refers to tea leaves and/or stem that have never been dried to a water content of less than 30% by weight, and usually have a water content in the range 60 to 90%.

### Expressing Juice

As used herein the term "expressing juice" refers to squeezing out juice from fresh tea leaves using physical force, as opposed to extraction of tea solids with the use of a solvent. Thus the term "expressing" encompasses such means as squeezing, pressing, wringing, spinning and extruding. It is possible that a small amount of solvent (e.g. water) is added to the fresh leaves during the expression step. However, in order to prevent significant extraction of tea solids by the solvent, the moisture content of the leaves during expression is that of fresh tea leaves as defined hereinabove. In other words, during the expression step, the moisture content of the tea leaves is between 30 and 90% by weight, more preferably between 60 and 90%. Furthermore, in order to avoid contamination of the tea juice, it is preferred that during the expression step the fresh tea leaves are not contacted with any non-aqueous solvents, such as alcohols.

### Beverage

As used herein the term "beverage" refers to a substantially aqueous drinkable composition suitable for human consumption.

### Beverage Precursor

A beverage precursor is defined as a fabricated composition suitable for preparing a beverage.

### Infusion package

As used herein, the term "infusion package" means a package comprising porous material. The porous material can be any material that is suitable for enabling water to infuse within the package without allowing any insoluble contents to leave the package, for example filter paper, nylon mesh, gauze, muslin, nonwoven fabric or some other similar material or fabric. Such infusion packages are well-known for use with leaf tea and include tea bags and tea pods. The preferred tea bags are those having a generally tetrahedral shape such as those disclosed in WO 97/20686 (Unilever), which is hereby incorporated by reference in its entirety.

### Leaf Size and Grade

For the purposes of the present invention, leaf particle size is characterised by sieve mesh size using the following convention:
- Tyler mesh sizes are used throughout.
- A "+" before the sieve mesh indicates the particles are retained by the sieve.
- A "-" before the sieve mesh indicates the particles pass through the sieve.

For example, if the particle size is described as -5 +20 mesh, then the particles will pass through a 5 mesh sieve (particles smaller than 4.0 mm) and be retained by a 20 mesh sieve (particles larger than 841 µm).

Leaf particle size may additionally or alternatively be characterized using the grades listed in the international standard ISO 6078-1982. These grades are discussed in detail in our European patent specification EP 1 365 657 B1 (especially paragraph [0041] and Table 2) which is hereby incorporated by reference.

### Catechins

As used herein the term "catechins" is used as a generic term for catechin, gallocatechin, catechin gallate, gallocatechin gallate, epicatechin, epigallocatechin, epicatechin gallate, epigallocatechin gallate, and mixtures thereof.

### Determination of Catechins and Caffeine in Leaf Tea

The amounts of catechins and caffeine in leaf tea are determined simultaneously by reverse-phase HPLC as follows:

### Sample Preparation

1. Grind the leaf tea using a Cyclotech^{™} 1093 sample mill (FOSS Ltd, Warrington, Cheshire, UK) fitted with a 0.5 µm screen, until a fine powder is achieved.
2. Weigh accurately approximately 200 mg of the ground tea into an extraction tube, and record the mass.
3. Warm at least 20 ml of a methanol-water solution (70% v/v methanol in distilled water) to 70°C.
4. Add 5 ml of the hot methanol-water solution to the extraction tube. Gently mix the methanol-water and tea material on a vortex mixer; place in a water bath at 70°C for 5 minutes; mix again and then place in a water bath at 70°C for a further 5 minutes.
5. Gently mix the methanol-water and tea material on a vortex mixer again and then allow too cool for a 10 minutes at an air temperature of 20°C.
6. Centrifuge the extraction tube at a relative centrifugal force (RCF) of 2900 g for 10 minutes.
7. The extraction tube should now contain a liquid supernatant on top of a plug of tea material. Carefully decant supernatant into a clean graduated test tube.
8. Add 5 ml of the hot methanol-water solution to the plug in the extraction tube. Gently mix the methanol-water and tea material on a vortex mixer; place in a water bath at 70°C for 5 minutes; mix again and then place in a water bath at 70°C for a further 5 minutes.
9. Gently mix the methanol-water and tea material on a vortex mixer again and then allow too cool for a 10 minutes at an air temperature of 20°C.
10. Centrifuge the extraction tube at a RCF of 2900 g for 10 minutes.
11. The extraction tube should now contain a liquid supernatant on top of a plug of tea material. Carefully decant supernatant into the graduated test tube containing the supernatant from step 7.
12. Make up the pooled supernatants to 10 ml with the methanol-water solution.
13. Add 1 ml of a solution of 2.5 mg/ml EDTA and 2.5 mg/ml ascorbic acid in distilled water to the graduated test tube.
14. Dilute 1 part of the pooled supernatant mixture with 4 parts (by volume) of 10% acetonitrile stabiliser solution (10% v/v acetonitrile, 0.25 mg/ml ascorbic acid and 0.25 mg/ml EDTA in distilled water).
15. Decant the diluted pooled supernatant mixture into microcentrifuge tubes and centrifuge in a bench top centrifuge at a RCF of 14000 g for 10 minutes.

### HPLC Analysis conditions

| | | | |
|---|---|---|---|
| **Column:** | Luna Phenyl hexyl 5µ, 250 x 4.60 mm | | |
| **Flow rate:** | 1 ml/min | | |
| **Oven temperature:** | 30°C | | |
| **Solvents:** | A: 2% acetic acid in acetonitrile | | |
| | B: 2% acetic acid and 0.02 mg/ml EDTA in water | | |
| **Injection volume:** | 10 µl | | |

| **Gradient:** | | | |
|---|---|---|---|
| Time | % Solvent A | % Solvent B | Step |
| 0 to 10 min | 5 | 95 | Isocratic |
| 10 to 40 min | 5 - 18 | 95 - 85 | Linear gradient |
| 40 to 50 min | 18 | 82 | Isocratic |
| 50 to 55 min | 50 | 50 | Wash |
| 55 to 75 min | 5 | 95 | Isocratic |

**Quantification:** Peak area relative to a calibration curve constructed daily. Calibration curve is constructed from caffeine and the concentration of catechins is calculated using the relative response factors of the individual catechins to caffeine (from the ISO catechin method - ISO/CD 14502-2). Individual caffeine standards (Sigma, Poole, Dorset, UK) are used as peak identification markers.

Determination of Catechins and Caffeine in a Beverage Produced by Contacting 2 g Leaf Tea with 200 ml Water at 90°C for 2 Minutes The amounts of catechins and caffeine delivered by a leaf tea are determined simultaneously by reverse-phase HPLC as follows:

### Sample Preparation

1. The leaf tea is removed from any package and 2 g is placed in a 500 ml container.
2. 1 litre of deionised water is then brought to boiling and 200 g immediately added to the 500 ml container.
3. The container is stored at an air temperature of 20°C and the leaf tea is allowed to statically infuse in the water.
4. After 2 minutes, the infusion is stirred for 5 s by hand using a spoon and the leaf tea is then immediately removed from the infusion by straining the contents of the container through muslin.
5. 9 ml of the liquid are then taken and 1.12 ml of acetonitrile added, along with 1.12 ml of a solution of 2.5 mg/ml EDTA and 2.5 mg/ml ascorbic acid in distilled water.
6. The resulting solution is then decanted into microcentrifuge tubes and centrifuged at a RCF of 14000 g for 10 minutes.

### HPLC Analysis conditions

The HPLC analysis conditions are identical to those given above for the leaf tea.

### SUMMARY OF THE INVENTION

We have surprisingly found that green leaf tea manufactured from the residue which remains after expressing greater than 300 ml juice per kg of fresh tea leaves has an increased efficiency of catechin delivery compared with conventional leaf tea or leaf made from leaf residue which remains after expressing less than 300 ml juice per kg.

Thus in a first aspect, the present invention provides a process comprising the steps of:
a) expressing juice from fresh tea leaves thereby to produce leaf residue and tea juice wherein the amount of expressed juice is greater than 300 ml per kg of fresh tea leaves;
b) subjecting the fresh tea leaves and/or the leaf residue to an enzyme deactivation treatment thereby to prevent fermentation of the leaf residue; and
c) drying the leaf residue to produce leaf tea.

Without wishing to be bound by theory, we believe that the damage caused by the expression process changes the morphology/microstructure of the leaf such that although the leaf residue has a reduced total level of infusible solids, the solids more easily infuse from the leaf into an infusion liquor.

In a second aspect the present invention provides a leaf tea obtained and/or obtainable by the process according to the first aspect.

In a further aspect the present invention provides a method of making a beverage using the leaf tea.

### BRIEF DESCRIPTION OF THE DRAWINGS

The invention will be described with reference to the accompanying drawings in which:
Figure 1 is a transmission electron micrograph of leaf tea made from unpressed leaves;
Figure 2 is a transmission electron micrograph of leaf tea made from the residue obtained by pressing 240 ml kg⁻¹ of juice; and
Figure 3 is a transmission electron micrograph of leaf tea made from the residue obtained by pressing 500 ml kg⁻¹ of juice.

### DETAILED DESCRIPTION

### Expression of Juice

Step (a) of the process of the invention comprises expressing juice from fresh tea leaves.

We have found that increasing the amount of expressed juice increases the efficiency of catechin delivery of the leaf tea manufactured from the leaf residue. Thus the amount of expressed juice is at least 300 ml per kg of the fresh tea leaves, preferably at least 350 ml, more preferably at least 400 ml and most preferably from 450 to 750 ml. When referring to the volume of juice expressed per unit mass of tea leaves it should be noted that the mass of the tea leaves is expressed on an "as is" basis and not a dry weight basis. Thus the mass includes any moisture in the leaves.

The expression step can be achieved in any convenient way so long as it allows for separation of the tea juice from the leaf residue and results in the required quantity of juice. The machinery used to express the juice may, for example, include a hydraulic press, a pneumatic press, a screw press, a belt press, an extruder or a combination thereof. Most preferably the juice is expressed by passing the fresh leaves through a screw press.

The juice may be obtained from the fresh leaves in a single pressing or in multiple pressings of the fresh leaves. Preferably the juice is obtained from a single pressing as this allows for a simple and rapid process.

In order to minimise degradation of the valuable tea compounds, it is preferred that the expression step is performed at ambient temperature. For example, the leaf temperature may be from 5 to 40°C, more preferably 10 to 30°C.

The time and pressure used in the expression step can be varied to yield the required amount of juice. Typically, however, the pressures applied to express the juice will range from 0.5 MPa (73 psi) to 10 MPa (1450 psi). The time over which the pressure is applied will typically range from 1 s to 1 hour, more preferably from 10 s to 20 minutes and most preferably from 30 s to 5 minutes.

Prior to expression, the fresh tea leaves may undergo a pretreatment including, for example, a unit process selected from maceration, withering or a combination thereof. In particular, maceration prior to expression may help in decreasing the time and/or pressure required to express the desired quantity of juice.

### Enzyme Deactivation

Step (b) of the process of the invention comprises subjecting the fresh tea leaves and/or the leaf residue to an enzyme deactivation treatment thereby to prevent fermentation of the leaf residue.

In one embodiment, the leaf residue is subjected to the enzyme deactivation step immediately following expression. In particular it is preferred that the leaf residue is subjected to the enzyme deactivation step not later than 10 minutes after expression, more preferably not later than 5 minutes and most preferably not later than 1 minute. This embodiment is particularly preferred if the tea juice is to be further processed after expression to yield an at least partially fermented tea product, i.e. if the tea juice is to be processed to make an oolong or black tea product.

In an alternative embodiment, the fresh tea leaves are subjected to the enzyme deactivation step before the expression step (a). This embodiment is particularly preferred if tea juice is to be further processed after expression to yield a substantially unfermented tea product, i.e. if the tea juice is to be processed to make a green tea product.

Any known treatment capable of enzyme denaturation may be used to deactivate the fermentation enzymes. A particularly convenient enzyme deactivation treatment is a heat treatment. For example, the fresh leaves may be steamed and/or pan-fried; and/or the leaf residue may be steamed and/or pan-fried.

Deactivation of the fermentation enzymes in the leaf residue allows the leaf residue to processed to produce green leaf tea.

### Processing the Leaf Residue

Step (c) of the process of the invention comprises drying the leaf residue to produce leaf tea. The leaf tea has a high efficiency of catechin delivery even though it has had the juice removed therefrom. Thus the leaf residue is processed separately from the tea juice. In particular the expressed tea juice is not contacted with the leaf residue during manufacture of the leaf tea.

The manufacturing processes of green leaf tea is well known and suitable processes are described, for example, in "Tea: Cultivation to Consumption", K.C. Willson and M.N. Clifford (Eds), 1st Edn, 1992, Chapman & Hall (London), Chapter 13.

A step common to manufacture of all leaf teas is a drying step. Efficient drying requires high temperatures and so it is preferred that step (c) of the process comprises drying the leaf residue at a temperature of at least 75°C, more preferably at least 90°C.

Expressing juice from the tea tends to reduce the particle size of leaf tea made there-from. This may, in part explain the enhanced infusion performance of the leaf teas made using the process of the present invention. Preferably therefore, at least 95% (more preferably from 98 to 100%) by weight of the leaf tea has a maximum linear dimension of less than 2 mm. In particular, it is preferred that at least 95% (more preferably from 98 to 100%) by weight of the leaf tea has a mesh size below 9 (i.e. passes through a Tyler Mesh of No. 9). Additionally or alternatively, at least 50% by weight of the leaf tea preferably has a particle size of -10 +40 mesh.

The leaf tea preferably comprises a high level of catechins, thus it is preferred that the leaf tea comprises at least 10% catechins by dry weight of the leaf tea, more preferably at least 12% and most preferably from 13 to 25%.

It is also preferred that the leaf tea comprises significant amounts of caffeine as caffeine has been shown to assist in body management, especially in weight control and/or control of body shape. Thus it is preferred that the leaf tea comprises caffeine in an amount of at least 1% by dry weight of the leaf tea, more preferably at least 2% and most preferably at least 2.5%. Too much caffeine, however, may result in an unpalatable beverage and/or unwanted physiological effects. Thus it is also preferred that the leaf tea comprises less than 5% caffeine by dry weight of the leaf tea, more preferably less than 4% and most preferably less than 3.5%.

The leaf tea produced by the process has a high efficiency of delivery of catechins to infusions prepared therefrom. It is preferred that the efficiency of catechin delivery is such that contact of 2 g of leaf tea with 200 ml water for 2 minutes at 90°C is sufficient to produce a beverage comprising catechins in an amount of at least 0.07% by weight of the beverage, more preferably at least 0.08% and most preferably at least 0.09%. The efficiency of catechin delivery should not be too high, however, otherwise the taste and/or appearance of the beverage may be impaired. Therefore it is preferred that contact of 2 g of the leaf tea with 200 ml water for 2 minutes at 90°C produces a beverage comprising catechins in an amount of less than 2.5% by weight of the beverage, more preferably less than 1% and most preferably less than 0.2%.

The leaf tea produced by the process preferably also has a high efficiency of delivery of caffeine to infusions prepared therefrom, as caffeine is also purported to be beneficial in terms of body management. Thus it is preferred that the efficiency of caffeine delivery is such that the beverage produced by contacting 2 g of the leaf tea with 200 ml water for 2 minutes at 90°C, comprises caffeine in an amount of between 0.005 and 0.5% by weight of the beverage, more preferably between 0.01 and 0.1% and most preferably between 0.02 and 0.07%.

### Packaging the Leaf Tea

In a preferred embodiment the process of the invention comprises a step (d) of packaging the leaf tea in an infusion package. The resulting beverage precursor comprises an infusion package containing green leaf tea with the morphology/microstructure and infusion properties afforded by the expression process. The leaf tea has a high efficiency of catechin delivery even though the juice has been removed there-from, thus it is preferred that the tea juice is not packaged in the infusion package.

The mass of leaf tea required to deliver suitable amounts of catechins to a beverage will depend upon the catechin content of the leaf tea and on the morphology of the leaf tea. It is preferred, however, that the mass of leaf tea packaged in the infusion package is at least 0.5 g, as smaller amounts are difficult to accurately portion and dose. More preferably the mass is at least 0.7 g, and most preferably at least 0.9 g. Preferably also, the mass of leaf tea is less than 5 g as larger amounts become inconvenient to store and/or handle. More preferably the mass is less than 4 g, most preferably less than 3 g.

The leaf tea is preferable packaged in the infusion package in combination with food-grade additive. The food-grade additive may be any edible material and may, for example, comprise saccharide (including sugars, oligosaccharides and/or polysaccharides), salt, sweetener (including artificial sweeteners such as aspartame, sucralose, and/or acesulfame K), protein, milk powder, food acid (and/or a salt thereof), flavour or a mixture thereof. Particularly preferred are sugars, oligosaccharide, sweetener, salt and mixtures thereof, owing to their ability to mask the bitterness of catechins.

### Use of the Leaf Tea

In a further aspect, the present invention provides a method of manufacturing a beverage comprising contacting the leaf tea obtained from the process of the invention with an aqueous medium. Suitable amounts of aqueous medium range from 50 g to 1000 g, more preferably 150 g to 500 g, most preferably 175 g to 300 g. The aqueous medium preferably comprises at least 90% water by weight of the aqueous medium, more preferably at least 98%, most preferably from 99.8 to 100%.

The leaf tea of this invention and/or the beverage made there-from may be used as a medicament or in the preparation of a medicament. In particular, the leaf tea and/or beverage may be used to provide any of the benefits associated with consumption of catechins such as treating and/or preventing cancer; and/or treating and/or preventing cardio-vascular disease. It is particularly preferred to use the leaf tea and/or beverage for controlling the bodyweight and/or shape of an individual. For example, the leaf tea or beverage may be used in a method of providing at least one of these benefits to an individual, the method comprising administering to the individual the beverage. Preferably the beverage is administered orally.

### Processing the Juice

Tea juice separated from the leaf residue typically has a high content of water-soluble tea solids and is a valuable raw material for producing tea products. Thus in a preferred embodiment, the juice is processed to produce a tea product.

The juice may be used to produce a green tea product, an oolong tea product or a black tea product. In the case of an oolong tea product or a black tea product then the juice is subjected to a fermentation step after expression.

### Diluting to Make a Beverage

In one embodiment the tea juice is diluted to produce a beverage. A suitable process is described, for example, in CN 1 718 030 A (LANCANGJIANG BEER ENTPR GROUP).

The juice is preferably diluted with an aqueous medium, preferably water. The beverage typically comprises at least 85% water, more preferably at least 90%, optimally between 95 and 99.9% by weight of the beverage.

Because the juice is relatively rich in tea solids, it can be diluted many-fold whilst still imparting tea-qualities to the resulting beverage. Preferably, therefore, the juice is diluted by at least a factor of 2 to produce the beverage (i.e. 1 part of juice is combined with 1 part diluent by weight). More preferably the juice is diluted by a factor of at least 5 (i.e. 1 part of juice is combined with 4 parts diluent by weight) and most preferably by a factor of at least 7.

The juice can be used to make concentrated beverages with high levels of tea solids. For example, the juice may be diluted by a factor of less than 50, more preferably less than 25 and most preferably less than 15.

The mass of a single serve of the beverage may be, for example, less than 600 g, more preferably less than 350 g, more preferably still less than 250 g and most preferably from 20 to 150 g.

The pH of the beverage may, for example, be from 2.5 to 8, more preferably 3 to 6, most preferably from 3.5 to 6. The beverage may comprise a food grade acid and/or salt thereof such as citric, malic, ascorbic acid or a mixture thereof.

The beverage preferably comprises at least one nutrient selected from carbohydrate, protein, fat, vitamins, minerals and mixtures thereof. The beverage may be low calorie (e.g. have an energy content of less than 100 kCal per 100 g of the beverage) or may have a high calorie content (e.g. have an energy content of more than 100 kCal per 100 g of the beverage, preferably between 150 and 1000 kCal). It is most preferred that the beverage is very low calorie such that a single serving has a total energy content of less than 5 kCal, more preferably still less than 1 kCal.

The beverage may also comprise any of salt, sweetener, flavours, colours, preservatives, antioxidants or a mixture thereof.

The beverage is preferably packaged. The package will typically be a bottle, can, carton or pouch.

The beverage is preferably sanitised e.g. by pasteurisation or sterilisation.

### Drying the Juice

In one embodiment the tea juice is dried to produce a liquid concentrate or powder. Preferably the juice is dried to a moisture content of less than 80% by weight, more preferably less than 50% by weight, more preferably still less than 30% by weight and most preferably less than 10% by weight. Any suitable drying process may be used including spray drying, freeze drying, oven drying, tray drying, vacuum drying or a combination thereof.

The concentrate or powder may, for example, be diluted or dissolved to produce a beverage, used as a food additive and/or used as a starting material for producing other tea-derived materials.

### EXAMPLES

The present invention will be further described with reference to the following examples.

### Example 1

This Example demonstrates the processing of fresh leaf to produce green leaf tea and green tea juice.

### Production of Juice

Fresh Kenyan tea leaves (which had not been withered and had a catechin content of around 15% by dry weight) of *Camellia Sinensis* var. *assamica* were steamed for 60 seconds at ~100°C to inactivate endogenous enzymes and thus prevent fermentation. Steamed leaves, cooled to room temperature, were chopped using a vegetable cutter to yield chopped leaf of average size of around 0.5 to 1 cm².

The dhool was then separated into three batches - A, B and C.

The dhool of batch A was pressed using a hydraulic press (5 Tonnes applied to a 500 g mass of leaf inside a cylinder of diameter 160 mm, resulting in a downward pressure of 354 psi (2.44 MPa)) to express green tea juice. The yield of green tea juice was 24 ml/100 g dhool.

The dhool of batch B was pressed using a screw press (Vincent horizontal continuous press model CP4, Vincent Corp., Tampa, Florida, USA) operating with a back-pressure of 80 psi (0.55 MPa). The resulting yield of juice was 50 ml/100 g dhool.

The Dhool of batch C was not pressed.

### Processing of Tea Juice

Expressed green tea juice was freeze-dried to produce a powder which may find utility in ready-to-drink applications or as an ingredient (source of tea actives) for a variety of other products.

### Production of Leaf Tea

The three batches of dhool were each broken up by hand and then dried using a fluidized bed drier (ten minutes at an inlet air temperature of 120°C, followed by ten minutes at an inlet air temperature of 90°C) to obtain three batches of green leaf tea each with a moisture content of less than 9% by weight.

### Example 2

This example demonstrates the properties of the leaf teas produced in Example 1.

### Catechin Delivery of the Leaf Tea

The efficiency of catechin and caffeine delivery of each batch of leaf tea was determined by infusing 2 g of loose leaf tea in 200 ml water at 90°C for 2 minutes. The results are shown in table 1.

**TABLE 1**

| **Batch of Leaf** | **Amount of Juice Expressed (ml kg⁻¹ fresh leaves)** | **Conc. Of Catechins in Infusion (wt %)** | **Conc. Of Caffeine in Infusion (wt %)** |
|---|---|---|---|
| **A** | 240 | 0.056 | 0.019 |
| **B** | 500 | 0.096 | 0.025 |
| **C** | 0 | 0.064 | 0.020 |

These results demonstrate that green leaf tea made from residual leaf from which 240 ml kg⁻¹ juice was pressed (batch A) has a similar efficiency of catechin and caffeine delivery as leaf tea made from unpressed leaves (batch C). In contrast, green leaf tea made from residual leaf from which 500 ml kg⁻¹ juice was pressed (batch B) has an improved efficiency of catechin and caffeine delivery.

### Grade Profile of the leaf Teas

The particle size distribution (grade profile) of each batch of leaf tea was determined by sieving through a series of increasingly narrow screens. The results are shown in Table 2.

**TABLE 2**

| **Batch** | **Amount of leaf in each grade (wt%)** | | | | | |
|---|---|---|---|---|---|---|
| | **+5 Mesh** | **-5 +10 Mesh** | **-10 +14 Mesh** | **-14 +24 Mesh** | **-24 +40 Mesh** | **-40 Mesh** |
| **A** | 0.43 | 66.77 | 19.08 | 11.80 | 1.80 | 0.76 |
| **B** | 0.06 | 40.62 | 19.09 | 22.32 | 10.81 | 7.37 |
| **C** | 0.21 | 63.33 | 21.82 | 12.73 | 1.50 | 0.74 |

The grade profile of batch A appears very similar to that of batch C. The grade profile of batch B, however, is shifted to smaller particle sizes (higher mesh numbers).

### Microstructure of the leaf Teas

Samples of tea leaf were fixed in 2.5% glutaraldehyde in 0.1 M sodium cacodylate buffer for 3.5 hours. They were then washed in 3 changes of 0.1 M sodium cacodylate buffer (2 x 1 hour and the final change overnight). Secondary fixation was performed using 1% osmium tetroxide for 3 hours followed by 3 x 1 hour changes of distilled water. The samples were then overnight in 1% aqueous uranyl acetate before dehydration in a graded series of aqueous ethanol solutions (70%, 90%, 100%, 100% and 1005 ethanol) for 1 hours at each level. This was followed by 2 x 1 hour changes in acetone before transferring the samples into 50/50 resin/acetone mixture overnight. The samples were then transferred into 100% resin mix for 24 hours before embedding the samples into fresh resin and polymerising at 60 degrees C for 48 hours. 90-110 nm ultrathin sections were cut using a Reichert UltraCut E ultramicrotome before examination of the sections on a JEOL 2100 transmission electron microscope operating at 200kV. Representative micrographs were taken using a GATAN ultrascan 4K camera and these images captured on Digital Micrograph software.

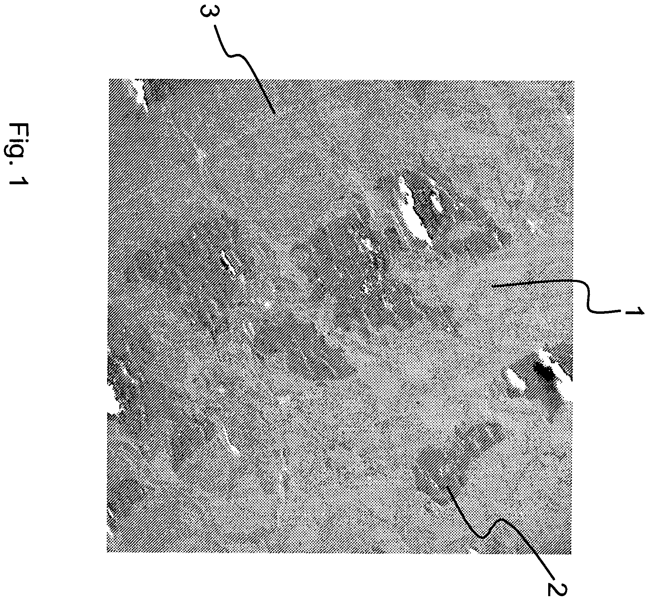

The results are shown in Figures 1 to 3 wherein the width of the area shown in each image is around 40 µm.
Figure 1 is a representative micrograph from the centre of the sample of leaf tea from batch C (unpressed leaf). Apparent from this micrograph are three types of feature, namely cell wall material (1), electron-dense intracellular material (2) and gaps (3) which are assumed to be vacuoles. The gaps (3) and intracellular material (2) appear to be distributed in regions of similar size and with no particular orientation.

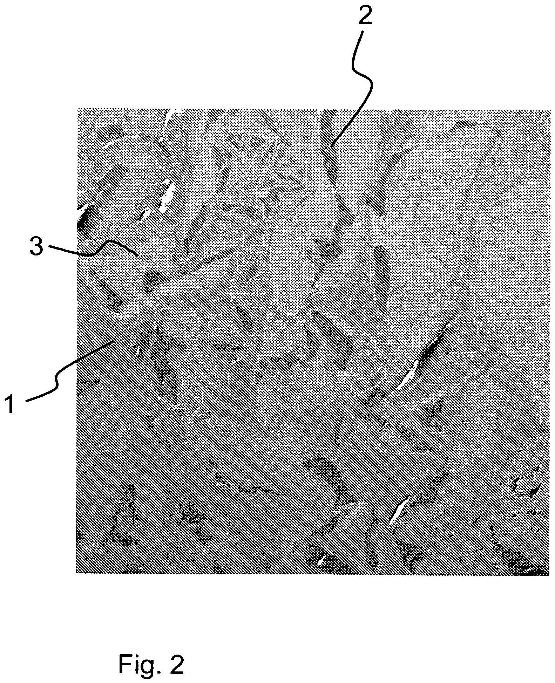

Figure 2 is a representative micrograph from the centre of the sample of leaf tea from batch A (240 ml juice expressed per kg of fresh leaves). It can be seen that the electron-dense intracellular material (2) is now in elongate regions which are generally aligned in a vertical direction (with respect to the way the micrograph is shown). In addition, a significant proportion of the micrograph in Figure 2 is still made up of gaps (3).

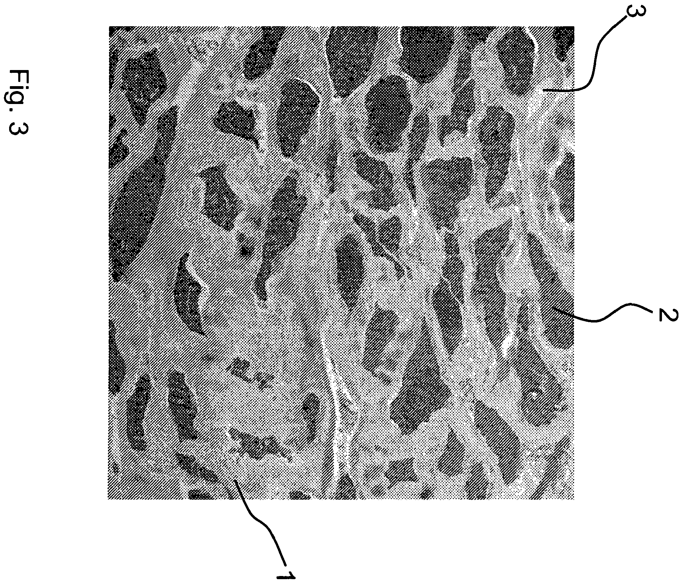

Figure 3 is a representative micrograph from the centre of the sample of leaf tea from batch B (500 ml juice expressed per kg of fresh leaves). As in Figure 2, the intra-cellular material (2) is in regions which are elongated and each is generally aligned in a common direction (in this case shown horizontally with respect to the way the micrograph is shown). In stark contrast to the micrograph shown in Figure 2, however, almost no gaps (3) are visible in the microstructure shown in Figure 3, which is presumably a result of the greater pressure exerted on batch B during pressing than that exerted on batch A.

## Claims

1. A process for manufacturing leaf tea comprising the steps of:
a) expressing juice from fresh tea leaves thereby to produce leaf residue and tea juice wherein the amount of expressed juice is greater than 300 ml per kg of fresh tea leaves;
b) subjecting the fresh tea leaves and/or the leaf residue to an enzyme deactivation treatment thereby to prevent fermentation of the leaf residue; and
c) drying the leaf residue to produce leaf tea.

2. A process according to claim 1 wherein the amount of juice expressed in step (a) is at least 350 ml per kg of fresh tea leaves.

3. A process according to claim 1 or claim 2 wherein the tea juice is diluted to produce a beverage.

4. A process according to claim 1 or claim 2 wherein the tea juice is dried to produce a liquid concentrate or powder.

5. A process according to any one of the preceding claims wherein at least 90% by weight of the leaf tea has a particle size below 9 mesh.

6. A process according to any one of the preceding claims, wherein the process comprises the additional step of:
d) packaging the leaf tea in an infusion package.

7. A process according to claim 6 wherein the tea juice is not packaged in the infusion package.

8. A process according to any one of the preceding claims wherein the moisture content of the fresh tea leaves from which juice is expressed in step (a) is from 30 to 90% by weight of the fresh tea leaves.

9. A leaf tea obtainable by the process according to any one of the preceding claims.

10. A leaf tea according to claim 9 wherein the infusion efficiency of the leaf tea is such that contact of 2 g of the leaf tea with 200 ml water for 2 minutes at 90°C produces a beverage comprising catechins in an amount of between 0.07% and 2.5% by weight of the beverage.

11. A leaf tea according to claim 9 or 10 wherein the leaf tea comprises catechins in an amount of at least 10% by weight of the leaf tea.

12. A leaf tea according to any one of claims 9 to 11 wherein the leaf tea comprises at least 90% by weight of material from *Camellia sinensis* var. *assamica.*

13. A method of manufacturing a beverage comprising contacting a leaf tea according to any one of claims 9 to 12 with an aqueous medium.

## Patentansprüche

1. Verfahren zur Herstellung von Blatttee, umfassend die Schritte:
a) Auspressen von Saft aus frischen Teeblättern, um dadurch Blattrückstand und Teesaft herzustellen, wobei die Menge an ausgepresstem Saft größer als 300 ml pro kg frischer Teeblätter ist;
b) Unterwerfen der frischen Teeblätter und/oder des Blattrückstands einer Enzymdesaktivierungsbehandlung, um dadurch eine Fermentation des Blattrückstandes zu verhindern, und
c) Trocknen des Blattrückstandes, um Blatttee herzustellen.

2. Verfahren gemäß Anspruch 1, wobei die Menge an in Schritt a) ausgepresstem Saft wenigstens 350 ml pro kg frischer Teeblätter ist.

3. Verfahren gemäß Anspruch 1 oder Anspruch 2, wobei der Teesaft unter Herstellung eines Getränks verdünnt wird.

4. Verfahren gemäß Anspruch 1 oder Anspruch 2, wobei der Teesaft getrocknet wird, um ein flüssiges Konzentrat oder Pulver herzustellen.

5. Verfahren gemäß einem der vorangehenden Ansprüche, wobei wenigstens 90 Gew.-% des Blatttees eine Partikelgröße von unter 9 mesh hat.

6. Verfahren gemäß einem der vorangehenden Ansprüche, wobei das Verfahren den zusätzlichen Schritt:
d) Verpacken des Blatttees in eine Aufgussverpackung
umfasst.

7. Verfahren gemäß Anspruch 6, wobei der Teesaft nicht in der Infusionsverpackung verpackt wird.

8. Verfahren gemäß einem der vorangehenden Ansprüche, wobei der Feuchtigkeitsgehalt der frischen Teeblätter, aus welchen in Schritt a) Saft ausgepresst wird, 30 bis 90 Gew.-% der frischen Teeblätter ist.

9. Blatttee, der durch das Verfahren gemäß einem der vorangehenden Ansprüche erhältlich ist.

10. Blatttee gemäß Anspruch 9, wobei die Aufgusseffizienz des Blatttees so ist, dass ein Kontakt von 2 g des Blatttees mit 200 ml Wasser mit 90 °C für 2 Minuten ein Getränk produziert, das Catechine in einer Menge von zwischen 0,07 und 2,5 Gew.-% des Getränks umfasst.

11. Blatttee gemäß Anspruch 9 oder 10, wobei der Blatttee Catechine in einer Menge von wenigstens 10 Gew.-% des Blatttees umfasst.

12. Blatttee gemäß einem der Ansprüche 9 bis 11, wobei der Blatttee wenigstens 90 Gew.-% Material aus *Camellia sinensis var. assamica* umfasst.

13. Verfahren zur Herstellung eines Getränks, umfassend In-Kontakt-Bringen eines Blatttees gemäß einem der Ansprüche 9 bis 12 mit einem wässrigen Medium.

## Revendications

1. Procédé de production de thé en feuilles, comprenant les étapes consistant à :
a) exprimer le jus de feuilles de thé fraîches pour produire ainsi un résidu de feuilles et un jus de thé, dans lequel la quantité de jus exprimé est supérieure à 300 ml par kg de feuilles de thé fraîches ;
b) soumettre les feuilles de thé fraîches et/ou le résidu de feuilles à un traitement de désactivation enzymatique, pour prévenir ainsi la fermentation du résidu de feuilles ; et
c) sécher le résidu de feuilles pour produire du thé en feuilles.

2. Procédé selon la revendication 1, dans lequel la quantité de jus exprimé à l'étape (a) est d'au moins 350 ml par kg de feuilles de thé fraîches.

3. Procédé selon la revendication 1 ou la revendication 2, dans lequel le jus de thé est dilué pour produire une boisson.

4. Procédé selon la revendication 1 ou la revendication 2, dans lequel le jus de thé est séché pour produire un concentré liquide ou une poudre.

5. Procédé selon l'une quelconque des revendications précédentes, dans lequel au moins 90 % en poids des feuilles de thé ont une taille de particules inférieure à 9 mesh.

6. Procédé selon l'une quelconque des revendications précédentes, dans lequel le procédé comprend l'étape supplémentaire de :
d) conditionner les feuilles de thé dans un conditionnement pour infusion.

7. Procédé selon la revendication 6, dans lequel le jus de thé n'est pas conditionné dans le conditionnement pour infusion.

8. Procédé selon l'une quelconque des revendications précédentes, dans lequel la teneur en humidité des feuilles de thé fraîches à partir desquelles le jus est exprimé à l'étape (a) est de 30 à 90 % en poids des feuilles de thé fraîches.

9. Thé en feuilles pouvant être obtenu par le procédé selon l'une quelconque des revendications précédentes.

10. Thé en feuilles selon la revendication 9, dans lequel l'efficience de l'infusion du thé en feuilles est telle que le contact de 2 g de feuilles de thé avec 200 ml d'eau pendant 2 minutes à 90° C produit une boisson comprenant des catéchines en une quantité entre 0,07 % et 2,5 % en poids de la boisson.

11. Thé en feuilles selon la revendication 9 ou 10, le thé en feuilles comprenant des catéchines en une quantité d'au moins 10 % en poids du thé en feuilles.

12. Thé en feuilles selon l'une quelconque des revendications 9 à 11, le thé en feuilles comprenant au moins 90 % en poids du matériau de *Camellia sinensis* var. *assamica.*

13. Méthode de production d'une boisson, comprenant la mise en contact d'un thé en feuilles selon l'une quelconque des revendications 9 à 12, avec un milieu aqueux.
